# EUROPÄISCHE PATENTSCHRIFT

(11) **EP 1 545 681 B1**
(45) Veröffentlichungstag und Bekanntmachung des Hinweises auf die Patenterteilung: **23.01.2008**
(21) Anmeldenummer: 03762595.1
(22) Anmeldetag: 02.07.2003
(51) Int. Cl.: A61M 25/06

(54) **KATHETEREINFÜHRVORRICHTUNG**
CATHETER INSERTION DEVICE
DISPOSITIF POUR INSERER UN CATHETER

(30) Priorität: 04.07.2002 DE 20210394 U
(43) Veröffentlichungstag der Anmeldung: 29.06.2005
(73) Patentinhaber: B. BRAUN MELSUNGEN AG, 34212 Melsungen (DE)
(72) Erfinder: WOEHR, Kevin, 34587 Felsburg (DE)
(74) Vertreter: Klingseisen, Franz
(86) Internationale Anmeldenummer: PCT/EP2003/007073
(87) Internationale Veröffentlichungsnummer: WO 2004/004819

(56) Entgegenhaltungen:
- EP-A- 0 554 841
- EP-A- 0 799 626
- DE-A- 3 210 148
- DE-A- 4 434 569
- DE-U- 20 104 539
- US-B1- 6 352 520

## Beschreibung

Die Erfindung betrifft eine Kathetereinführvorrichtung nach dem Oberbegriff des Anspruchs 1.

Eine Vorrichtung dieser Art ist aus DE 201 04 539 U und EP 1 003 588 bekannt, wobei in einer hohlen Katheterbuchse ein Nadelschutzelement angeordnet ist, das beim Zurückziehen der Hohlnadel aus dem Katheter über eine Eingriffseinrichtung nahe der Spitze der Hohlnadel mit dieser in Eingriff tritt und die Spitze abdeckt, wenn die Hohlnadel vom Katheter getrennt wird. Bei dieser Ausgestaltung kann nach dem Herausziehen der Hohlnadel aus dem Katheter durch diesen Blut austreten, mit dem das Bedienungspersonal in Kontakt kommen kann.

Aus DE 44 34 569 A ist eine Kathetereinführvorrichtung mit Blutdichtung bekannt, bei der eine scheibenförmige Dichtung mit einem rohrförmigen Ansatz in einen hohlzylindrischen Abschnitt eines Bauteils eingreift, das in die Katheterbuchse eingesetzt ist. Ein Teil des Bauteils liegt außerhalb der Katheterbuchse und ist mit einem Nadelschutzelement versehen. Bei einer Bauform mit Ventilscheibe ist diese stirnseitig an dem Bauteil angebracht. Beim Herausziehen der Nadel aus der Katheterbuchse wird das Nadelschutzelement zusammen mit dem Bauteil von der Katheterbuchse entfernt, sodass die Dichtung aus der Katheterbuchse mit herausbewegt wird. Hierdurch kann Blut aus der Katheterbuchse austreten. Dadurch, dass das Nadelschutzelement in der Bereitstellung außerhalb der Katheterbuchse angeordnet ist, ergibt sich ein in Achsrichtung sperriger Aufbau.

Der Erfindung liegt die Aufgabe zugrunde, eine Kathetereinführvorrichtung der eingangs angegebenen Art so auszubilden, dass ein Blutaustritt aus dem Katheter nach dem Entfernen der Hohlnadel mit Nadelschutzelement verhindert wird.

Diese Aufgabe wird erfindungsgemäß durch die Merkmale im kennzeichnenden Teil des Anspruchs 1 gelöst. Dadurch, dass in der Bereitstellung zwischen Katheter und Nadelschutzelement ein Rückschlagventil in der Katheterbuchse angeordnet ist, durch das sich die Hohlnadel erstreckt, kann nach dem Herausziehen der Hohlnadel aus dem Katheter dieser zuverlässig abgeschlossen werden, so dass ein Blutaustritt verhindert wird, während gleichzeitig die Spitze der Hohlnadel durch das Nadelschutzelement sicher abgedeckt wird, so dass sich die Bedienungsperson an der Nadelspitze nicht verletzen kann.

Beispielsweise Ausführungsformen der Erfindung werden nachfolgend anhand der Zeichnung näher erläutert. Es zeigen
- Fig. 1: einen Längsschnitt durch eine Kathetereinführvorrichtung in der Bereitstellung,
- Fig. 2: die Kathetereinführvorrichtung mit herausgezogener Hohlnadel,
- Fig. 3: die Vorrichtung mit aufgesteckter Spritze,
- Fig. 4: eine Schnittansicht längs der Linie A-A in Fig. 1,
- Fig. 5: einen Längsschnitt durch eine andere Ausführungsform,
- Fig. 6: eine Ansicht der Ventilscheibe, und
- Fig. 7: ein Ventilbetätigungselement in verschiedenen Ansichten,

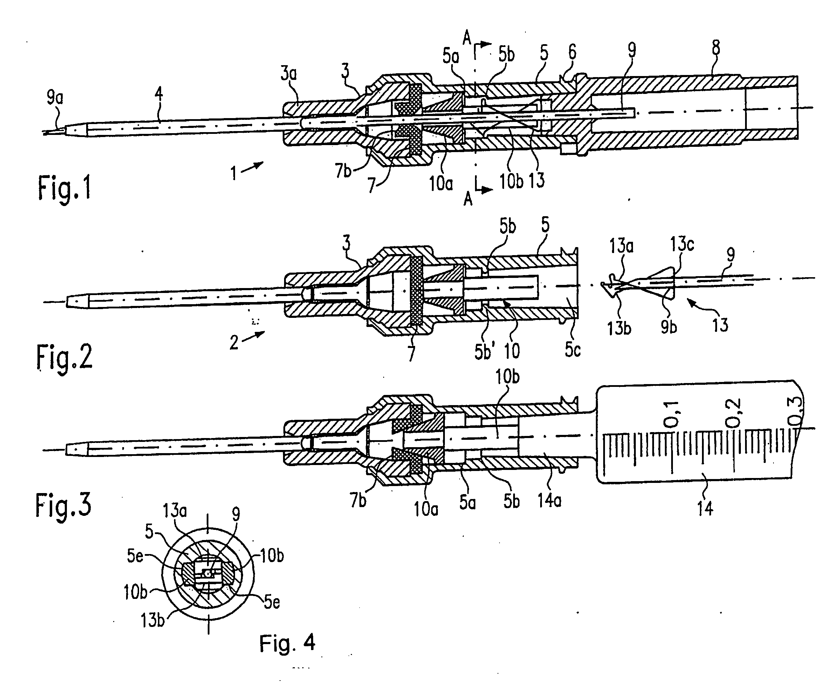

Fig. 1 zeigt eine Kathetereinführvorrichtung 1 mit einer Katheterbuchse 2, die bei dem dargestellten Ausführungsbeispiel zweiteilig ausgebildet ist. Ein distales Buchsenelement 3 der Katheterbuchse weist einen Halteabschnitt 3a auf, in dem ein Katheter 4 eingepresst ist. Das proximale Ende des Buchsenelementes 3 ist gegenüber dem distalen Ende im Durchmesser vergrößert und bildet einen Verbindungsabschnitt mit einem Buchsenelement 5, das mit dem distalen Ende das proximale Ende des Buchsenelements 3 übergreift und am proximalen Ende mit einem Luer-Gewinde 6 versehen ist. Zwischen den beiden Buchsenelementen 3 und 5 ist ein Rückschlagventil in Form einer Ventilscheibe 7 eingesetzt, die durch die beiden Buchsenelemente 3 und 5 in ihrer Lage fixiert ist.

In der Bereitstellung nach Fig. 1 ist in die Katheterbuchse 2 eine Nadelbuchse 8 eingesetzt, an der eine Hohlnadel 9 befestigt ist, die sich durch die Ventilscheibe 7 und den Katheter 4 erstreckt, so dass die Nadelspitze 9a frei liegt. Zwischen Nadelbuchse 8 und Ventilscheibe 7 ist im proximalen Buchsenelement 5 ein Ventilbetätigungselement 10 verschiebbar angeordnet, das einen kegelstumpfförmigen Anlageabschnitt 10a aufweist, der zum Öffnen der Ventilscheibe 7 dient, wie Fig. 3 zeigt. Auf der proximalen Seite schließt sich an den Anlageabschnitt 10a ein Stößelabschnitt 10b an, der einen Hohlraum zur Aufnahme eines Nadelschutzelementes 13 aufweist. Bei dem dargestellten Ausführungsbeispiel ist der Stößelabschnitt 10b durch zwei beabstandete Stößel ausgebildet, zwischen denen das Nadelschutzelement in Form eines Federclips 13 eingesetzt ist, wie dies die Querschnittsansicht in Fig. 4 zeigt.

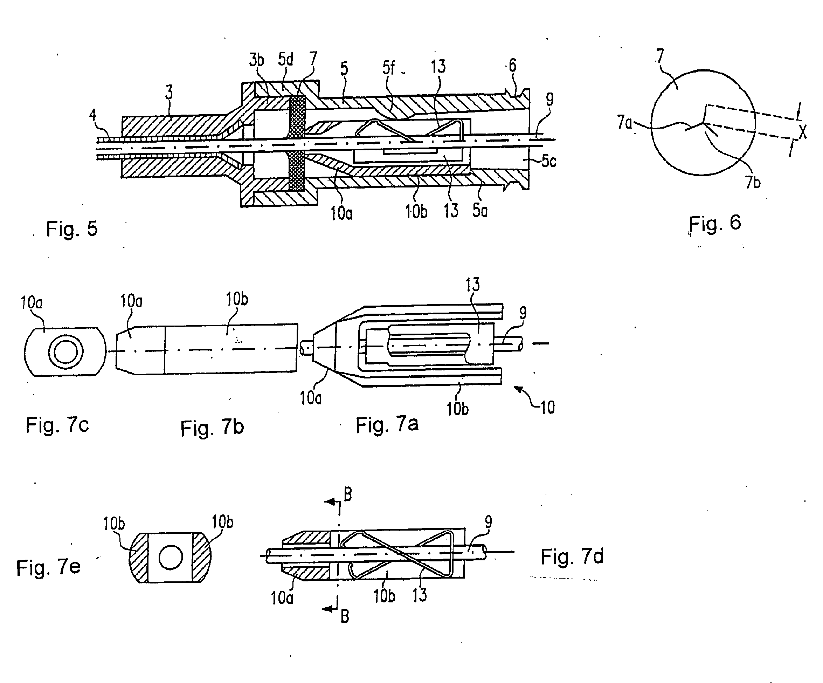

Wenn die Hohlnadel 9 aus der Katheterbuchse 2 zurückgezogen wird, kommt eine nahe der Nadelspitze 9a vorgesehene Eingriffseinrichtung 9b (Fig. 2) in Form eines radialen Vorsprungs an der Hohlnadel, der durch eine leichte Quetschung ausgebildet sein kann, mit dem Außenumfang einer Bohrung in der Rückwand 13c des Federclips 13 in Eingriff, so dass der Federclip 13 mit der Nadel 9 aus der Katheterbuchse gezogen wird, während sich gleichzeitig die Federarme 13a und 13b des Federclips um die Nadelspitze legen und diese vollständig abdecken und blockieren. In dieser in Fig. 2 wiedergegebenen Trennstellung schließt die Ventilscheibe 7 aufgrund ihrer Elastizität die Durchtrittsöffnung für die Hohlnadel 9, so dass durch den Katheter 4 kein Blut austreten kann. Die Ventilscheibe ist, wie Fig. 6 zeigt, beispielsweise mit drei von der Mitte ausgehenden und sich radial über einen kurzen Abschnitt X erstreckenden Einschnitten 7a versehen, die elastische Laschen 7b dazwischen bilden, welche durch die Hohlnadel aufgeweitet werden können.

Fig. 3 zeigt das Einsetzen einer Spritze 14 in die Katheterbuchse 2, wobei der Halsabschnitt 14a der Spritze an dem Stößelabschnitt 10b des Ventilbetätigungselementes 10 zum Anliegen kommt und dieses gegen die Ventilscheibe 7 drückt, so dass der kegelstumpfförmige Anlageabschnitt 10a die Laschen 7b der Ventilscheibe nach außen drückt und dadurch das Ventil öffnet, so dass eine Flüssigkeit aus der Spritze 14 in den Katheter 4 eingeführt werden kann.

Die Schräge des Kegelstumpfes am Anlageabschnitt 10a und der Verschiebeweg des Betätigungselementes 10 relativ zur Ventilscheibe 7 sind so ausgelegt, dass aufgrund der Elastizität des Materials der Ventilscheibe 7 die Laschen 7b den Anlageabschnitt 10a nach rechts in Fig. 3 drücken, wenn die Spritze 14 aus der Katheterbuchse 2 herausgezogen wird. Hierdurch wird die Ventilscheibe 7 selbsttätig geschlossen, wie dies die Stellung in Fig. 2 zeigt.

In dem Buchsenelement 5 ist durch einen Absatz 5a ein Anschlag für das Betätigungselement 10 ausgebildet, um dessen Position in der Trennstellung nach Fig. 2 zu definieren. Hierbei liegt der kegelstumpfförmige Anlageabschnitt 10a nahe an dem Anschlag 5a, während er mit dem distalen Ende an der Ventilscheibe 7 anliegt, wie dies Fig. 2 zeigt. Die radialen Schlitze 7a der Ventilscheibe 7 sind so ausgelegt, dass in der Bereitstellung nach Fig. 1 die Laschen 7b weniger radial aufgebogen werden als in der Öffnungsstellung durch den Anlageabschnitt 10a in Fig. 3.

Wie die Querschnittsansicht in Fig. 4 zeigt, sind die beiden Stößel 10b des Ventilbetätigungselementes 10 in Längsnuten 5e des Buchsenelementes 5 geführt und sie stehen radial nach innen in die Bohrung 5c des Buchsenelements 5 vor, so dass sie eine Anlagefläche für den Halsabschnitt 14a der Spritze 14 bilden. Die Bohrung 5c im Buchsenabschnitt 5 ist leicht konisch gestaltet entsprechend dem konischen Halsabschnitt 14a der Spritze.

Auf dem Innenumfang der Bohrung 5c des Buchsenelementes 5 ist ein weiterer Absatz 5b mit kleinerem Durchmesser ausgebildet, an dem die radial äußeren Bereiche der Federarme 13a und 13b in der Bereitstellung nach Fig. 1 anliegen. Hierdurch wird der Federclip 13 im Buchsenelement 5 in seiner Stellung fixiert. Wenn die Nadelbuchse 8 mit der Hohlnadel 9 aus der Katheterbuchse 2 herausgezogen wird, wird zunächst der Federclip 13 durch Anlage an dem Absatz 5b gehalten, bis der radiale Vorsprung 9b an der Rückwand 13c des Federclips zum Anliegen kommt. In dieser Stellung können die beiden Federarme 13a, 13b sich von dem Absatz 5b lösen und nach innen zum Abdecken der Nadelspitze zurückfedern, wie Fig. 2 zeigt, worauf der Federclip 13 mit der Hohlnadel 9 aus der Kathederbuchse herausgezogen werden kann.

Bei dem Ausführungsbeispiel nach den Fig. 1 bis 3 wird der distale Endabschnitt des Buchsenelementes 5 auf den proximalen Endabschnitt des Buchsenelementes 3 aufgeschrumpft, angeschweißt oder angeklebt, nachdem in das Buchsenelement 5 das Ventilbetätigungselement 10 und die Ventilscheibe 7 eingesetzt sind. Es ist auch möglich, die beiden Buchsenelemente 3 und 5 beipielsweise durch ein Gewinde miteinander zu verbinden, das nach der Montage gegen Lösen gesichert wird. Der Federclip 13 wird zusammen mit der Hohlnadel 9 bei der Montage in die Bohrung 5c des Buchsenelementes 5 eingesetzt, wobei die radial äußeren Bereiche der Federarme 13a, 13b unter elastischer Verformung am Absatz 5b einrasten.

Vorzugsweise kann vor dem Absatz 5b ein Wulst 5b' in der Bohrung 5c des Buchsenelementes ausgebildet sein, wie dies Fig. 2 zeigt. Hierdurch wird die Rast- und Haltewirkung des Federclips 13 verstärkt.

Fig. 5 zeigt eine abgewandelte Ausführungsform der Verbindung der beiden Buchsenelemente 3 und 5, bei der zwei zylindrische Abschnitte 3b und 5d ineinandergreifen. Es kann ein Gewinde zwischen diesen beiden zylindrischen Abschnitten vorgesehen werden. Es ist aber auch möglich, diese beiden Abschnitte zu verkleben oder zu verschweißen.

Bei dieser Ausführungsform ist auch das Ventilbetätigungselement 10 gegenüber der Ausführungsform nach den Fig. 1 bis 3 modifiziert. Fig. 7a zeigt eine Seitenansicht des etwa U-förmigen Betätigungselementes 10 mit dem darin eingesetzten Federclip 13. Wie die um 90° verdrehte Seitenansicht in Fig. 7b zeigt, ist der Anlageabschnitt l0a auf gegenüberliegenden Seiten teilweise abgeflacht, so dass sich die Breite der Stößelabschnitte 10b in den Anlageabschnitt 10a hinein erstreckt. Fig. 7c ist eine Stirnansicht von links in Fig. 7b und zeigt die abgeflachte Bauform des Anlageabschnitts 10a. Fig. 7d ist eine Schnittansicht längs der Mittellinie in Fig. 7b. Fig. 7e zeigt einen Schnitt durch das Ventilbetätigungselement 10 längs der Linie B-B in Fig. 7d.

Fig. 5 zeigt das Ventilbetätigungselement 10 in der unteren Hälfte in der Ansicht von Fig. 7a und in der oberen Hälfte in einer um 90° entsprechend Fig. 7b verdrehten Schnittansicht. Der Absatz 5a für die Positionierung des Ventilbetätigungselementes 10 im Buchsenelement 5 ist hierbei an den Enden der diametral gegenüberliegenden Nuten 5e (Fig. 4) ausgebildet, so dass die proximalen Enden der Stößelabschnitte 10b an den Absätzen 5a anliegen. Dem Absatz 5b in Fig. 1 bis 3 entspricht bei der Ausführungsform nach Fig. 5 ein an diametral gegenüberliegenden Stellen der Bohrung 5c des Buchsenelementes 5 nach innen vorstehender Wulst 5f, der am Buchsenelement 5 angeformt ist und zur Fixierung des Federclips 13 im Buchsenelement 5 dient, bis die Federarme 13a, 13b über die Nadelspitze nach innen federn und der Federclip mit der Hohlnadel 9 aus der Kathederbuchse herausgezogen wird.

Damit beim Herausziehen der Hohlnadel 9 aus der Katheterbuchse 2 der Federclip 13 in der Katheterbuchse gehalten wird, bis der radiale Vorsprung 9b an der Hohlnadel mit der Rückwand 13c zum Abdecken der Nadelspitze in Eingriff tritt, ist auf dem Innenumfang des proximalen Buchsenelementes 5 ein sich radial nach innen erstreckender Wulst 5f ausgebildet, an dem die radial äußeren Bereiche der Federarme 13a und 13b zum Anliegen kommen und den Federclip halten, bis die Federarme zum Abdecken der Spitze radial nach innen zurückfedern. Der Innendurchmesser des Wulstes 5f ist nur geringfügig kleiner ausgelegt als die maximale radiale Abmessung an den Federarmen 13a und 13b, so dass bei der Montage der Federclip 13 durch leichten Druck in die wiedergegebene Stellung in der Katheterbuchse gebracht werden kann.

Bei der Ausführungsform einer Kathetereinführvorrichtung nach den Fig. 1 bis 7 kann die Ventilscheibe 7 in der Stellung des Ventilbetätigungselementes 10 in Fig. 2 durch einen mittels der Spritze 4 erzeugten Unterdruck zum Absaugen von Flüssigkeit aus dem Katheter geöffnet werden.

Das Rückschlagventil in Form einer Ventilscheibe 7 wird zweckmäßigerweise aus elastischem Silikon gefertigt, während für die Buchsenelemente 3 und 5 sowie für das Ventilbetätigungselement 10 ein entsprechend steifer Kunststoff verwendet wird.

## Patentansprüche

1. Kathetereinführvorrichtung, umfassend
eine etwa hohlzylindrische Katheterbuchse (2), an deren distalem Ende ein Katheter (4) angebracht ist,
eine Nadelbuchse (8) mit einer daran angebrachten Hohlnadel (9), die sich in der Bereitstellung durch die Katheterbuchse (2) und den Katheter (4) erstreckt,
ein Nadelschutzelement (13), das auf der Nadel (9) verschiebbar in der Katheterbuchse (2) angeordnet ist und einen Eingriffsabschnitt (13a, 13b) aufweist, der mit einer auf dem Innenumfang der Katheterbuchse (2) ausgebildeten Eingriffseinrichtung in Form einer Durchmesseränderung (5b, 5f) der Katheterbuchse (5) in der Bereitstellung in Eingriff steht und der mit der Nadel nahe der Nadelspitze in Eingriff tritt, wenn die Hohlnadel aus der Katheterbuchse (2) herausgezogen wird,
**gekennzeichnet**
**durch** ein Rückschlagventil (7), das in der Katheterbuchse (2) zwischen Katheter (4) und Nadelschutzelement (13) gehalten ist, wobei sich die Hohlnadel (9) in der Bereitstellung durch das Rückschlagventil erstreckt, das sich nach Herausziehen der Nadel selbsttätig schließt, und
**durch** ein Ventilbetätigungselement (10), das in der Katheterbuchse (2) verschiebbar geführt ist und einen Hohlraum zur Aufnahme des Nadelschutzelementes (13) in der Bereitstellung aufweist.

2. Vorrichtung nach Anspruch 1, wobei die Katheterbuchse (2) zweiteilig ausgebildet ist und das Rückschlagventil (7) zwischen einem distalen Buchsenelement (3) und einem proximalen Buchsenelement (5) gehalten wird, die miteinander verbunden sind.

3. Vorrichtung nach Anspruch 1, wobei das Ventilbetätigungselement (10) wenigstens einen Stößel (10b) aufweist, der sich in axialer Richtung erstreckt.

4. Vorrichtung nach einem der vorhergehenden Ansprüche, wobei das Ventilbetätigungselement (10) mit einem kegelstumpfförmigen distalen Endabschnitt (10a) ausgebildet ist.

5. Vorrichtung nach Anspruch 1, wobei die Eingriffseinrichtung (5) auf dem Innenumfang der Katheterbuchse (2) als radialer Vorsprung (5b) ausgebildet ist.

6. Vorrichtung nach Anspruch 1, wobei das Rückschlagventil (7) in der Form einer Ventilscheibe mit wenigstens einem radialen Schlitz (7a) ausgebildet ist, der sich von der Mitte der Ventilscheibe aus radial nach außen erstreckt.

7. Vorrichtung nach einem der vorhergehenden Ansprüche, wobei die Nadel (9) eine Eingriffseinrichtung für das Nadelschutzelement (13) in Form eines radialen Vorsprungs (9b) aufweist.

8. Vorrichtung nach einem der vorhergehenden Ansprüche, wobei das Nadelschutzelement als Federclip (13) ausgebildet ist, der ausgehend von einer mit einer Bohrung versehenen Rückwand (13c) diametral gegenüberliegende Federarme (13a, 13b) aufweist, die mit abgebogenen Endabschnitten die Nadelspitze übergreifen und blockieren, wenn die Eingriffseinrichtung (9b) der Nadel an der Rückwand (13c) zum Anliegen kommt.

## Claims

1. Catheter insertion device comprising
an approximately hollow-cylindrical catheter hub (2) to whose distal end a catheter (4) is attached,
a needle hub (8) having a hollow needle (9) attached thereon and extending in the ready position through the catheter hub (2) and the catheter (4),
a needle guard element (13) arranged displaceably on the needle (9) in the catheter hub (2) and having an engaging section (13a, 13b) which is engaged with an engaging means formed on the inner circumference of the catheter hub (2) in the form of a diameter variation (5b, 5f) in the catheter hub (2) in the ready position, and engages with the needle near the needle tip when the hollow needle is removed from the catheter hub (2),
**characterized**
**by** a check valve (7) held in the catheter hub (2) between the catheter (4) and the needle guard element (13), wherein the hollow needle (9) extends in the ready position through the check valve, which closes automatically after the removal of the needle, and
by a valve actuating element (10) displaceably guided in the catheter hub (2) and having a hollow space for receiving the needle guard element (13) in the ready position.

2. Device according to claim 1, wherein the catheter hub (2) has a two-part form and the check valve (7) is held between a distal hub element (3) and a proximal hub element (5) which are joined to one another.

3. Device according to claim 1, wherein the valve actuating element (10) has at least one plunger (10b) extending in the axial direction.

4. Device according to one of the preceding claims, wherein the valve actuating element (10) is formed with a truncated cone-shaped distal end section (10a).

5. Device according to claim 1, wherein the engaging means (5) is formed on the inner circumference of the catheter hub (2) as a radial projection (5b).

6. Device according to claim 1, wherein the check valve (7) is formed in the shape of a valve disc with at least one radial slit (7a) extending from the centre of the valve disc radially outward.

7. Device according to one of the preceding claims, wherein the needle (9) has an engaging means for the needle guard element (13) in the shape of a radial projection (9b).

8. Device according to one of the preceding claims, wherein the needle guard element is formed as a spring clip (13) which has diametrically opposite spring arms (13a, 13b) starting from a rear wall (13c) provided with a bore, wherein bent end sections of the spring arms (13a, 13b) overlap and block the needle tip when the engaging means (9b) of the needle comes to abut on the rear wall (13c).

## Revendications

1. Dispositif pour insérer un cathéter, comprenant
une douille de cathéter sensiblement cylindrique creuse (2), sur l'extrémité distale de laquelle est fixé un cathéter (4),
une douille à aiguille (8) avec une aiguille creuse insérée dans celle-ci (9) qui s'étend, lors de la mise en place, à travers la douille de cathéter (2) et le cathéter (4),
un élément de protection d'aiguille (13) qui est disposé sur l'aiguille (9) de façon déplaçable dans la douille de cathéter (2) et présente une section d'attaque (13a, 13b), qui se trouve dans la mise à disposition en attaque avec un dispositif d'attaque formé sur la périphérie interne de la douille de cathéter (2) sous forme d'une modification de diamètre (5b, 5f) de la douille de cathéter (5) et qui vient en prise avec l'aiguille proche de la pointe lorsque l'aiguille creuse est sortie de la douille de cathéter (2),
**caractérisé par**
une soupape antiretour (7) qui est maintenue dans la douille de cathéter (2) entre le cathéter (4) et l'élément de protection d'aiguille, de telle sorte que l'aiguille creuse (9) dans la position de mise à disposition s'étend à travers la soupape antiretour qui, après avoir sorti l'aiguille, se ferme automatiquement, et
par un élément d'actionnement de soupape (10) qui est guidé de façon déplaçable dans la douille de cathéter (2) et qui présente un espace creux pour recevoir l'élément de protection d'aiguille (13) dans la position de mise à disposition.

2. Dispositif selon la revendication 1, dans lequel la douille de cathéter (2) est constituée de deux parties et la soupape antiretour (7) est maintenue entre un élément de douille distal (3) et un élément de douille proximal (5) qui sont reliés ensemble.

3. Dispositif selon la revendication 1, dans lequel l'élément d'actionnement de soupape (10) présente au moins un coulisseau (10b) qui s'étend dans la direction axiale.

4. Dispositif selon l'une des revendications précédentes, dans lequel l'élément d'actionnement de soupape (10) est formé par une section d'extrémité distale en forme de cône tronqué (10a).

5. Dispositif selon la revendication 1, dans lequel le dispositif d'attaque (5) est formé sur la périphérie interne de la douille de cathéter (2) en tant que saillie radiale (5b).

6. Dispositif selon la revendication 1, dans lequel la soupape antiretour (7) se présente sous la forme d'un disque de soupape avec au moins une fente radiale (7a) qui s'étend depuis le milieu du disque de soupape radialement vers l'extérieur.

7. Dispositif selon l'une des revendications précédentes, dans lequel l'aiguille (9) présente un dispositif d'attaque pour l'élément de protection d'aiguille (13) sous forme d'une saillie radiale (9b).

8. Dispositif selon l'une des revendications précédentes, dans lequel l'élément de protection d'aiguille est conçu en tant que clip à ressort (13) qui, en partant d'une paroi arrière (13c) munie d'un alésage, présente des bras à ressort (13a, 13b) diamétralement opposés qui recouvrent la pointe d'aiguille par des sections d'extrémité recourbées et exercent une action de blocage lorsque le dispositif d'attaque (9b) de l'aiguille vient en appui contre la paroi arrière (13c).
